# EUROPEAN PATENT SPECIFICATION

(11) **EP 2 106 735 B1**
(45) Date of publication and mention of the grant of the patent: **25.02.2015**
(21) Application number: 09004842.2
(22) Date of filing: 01.04.2009
(51) Int. Cl.: A61B 1/005

(54) **Endoscope configured to be carried with hand**
Endoskop, das dazu eingerichtet ist, in der Hand getragen zu werden
Endoscope configuré pour être portable

(30) Priority: 01.04.2008 JP 2008095502
(43) Date of publication of application: 07.10.2009
(73) Proprietor: Olympus Medical Systems Corp., Tokyo 151-0072 (JP)
(72) Inventor: Watanabe, Katsushi, Hachioji-shi Tokyo 192-8512 (JP); Ogawa, Tomoaki, Hachioji-shi Tokyo 192-8512 (JP); Okada, Takeshi, Hachioji-shi Tokyo 192-8512 (JP); Nemoto, Shigeru, Hachioji-shi Tokyo 192-8512 (JP); Imai, Shunichi, Hachioji-shi Tokyo 192-8512 (JP); Suzuki, Takeo, Hachioji-shi Tokyo 192-8512 (JP)
(74) Representative: von Hellfeld, Axel

(56) References cited:
- EP-A- 1 719 445
- EP-A- 1 836 949
- EP-A2- 0 668 052
- JP-A- 2000 189 385
- JP-A- 2002 282 199
- JP-A- 2005 237 818
- US-A1- 2002 077 528
- US-A1- 2002 137 987
- US-A1- 2002 184 122
- US-A1- 2003 004 460
- US-A1- 2003 009 086
- US-A1- 2006 287 575
- US-A1- 2007 185 379

## Description

The present invention relates to an endoscope configured to be carried with the hand with a vertical hold and a horizontal hold, wherein an antenna is connected to one side of the horizontal hold. The antenna extends so as to be inclined distally in the longitudinally axial direction of the endoscope and outwardly in the orthogonal direction to the axial direction.

An endoscope includes an insertion portion extending in the longitudinally axial direction and configured to be inserted into the interior of the body. A bending portion is provided in the distal end portion of the insertion portion and configured to be actuated to be bent. An operation portion is coaxially coupled to the proximal end portion of the insertion portion, extends in the longitudinally axial direction, and is configured to be held and operated by an operator. In the operation portion, a grasp portion extends in the longitudinally axial direction and configured to be grasped by an operator. In the operation portion, a bending operation portion is also provided on the proximal end side with respect to the grasp portion, and the bending operation portion is configured to operate the bending portion to be bent. When the endoscope is used for a lying patient, the endoscope is normally used with a vertical hold. That is, the grasp portion is grasped so as to be enclosed with one hand, and the thumb of the one hand is placed on the bending operation portion. Then, while the endoscope held with the one hand is arranged upright along a vertical direction, the endoscope is appropriately moved and the bending operation portion is operated with the thumb of the one hand holding the endoscope.

Jpn. Pat. Appln. KOKAI Publication Nos. 2002-282199 and 2005-237818 disclose endoscopes used with the vertical hold. In the endoscope disclosed in Jpn. Pat. Appln. KOKAI Publication No. 2002-282199, in the operation portion, the bending operation knob as the bending operation portion is provided on the one widely directional side wherein a wide direction is orthogonal to the longitudinally axial direction. A hook shaped part protrudes towards the other widely directional side from the other widely directional side of the proximal end portion of the operation portion. The terminal end portion of the hook shaped part is curved toward the longitudinally axially distal end side. When using the endoscope with the vertical hold, the hook shaped portion covers the hand and the hook shaped portion is supported by the hand grasping a trunk portion of the operation portion as the grasp portion, and therefore the endoscope is stably held. In the endoscope disclosed in Jpn. Pat. Appln. KOKAI Publication No. 2005-237818, a trackball as the bending operation portion is provided on the one widely directional side of the proximal end portion of the operation portion. A U-shaped hook protrudes toward the other widely directional side from the other widely directional side of the operation portion. When using the endoscope with the vertical hold, some of the thumb and the fingers of the hand grasping the grasp portion are inserted through the hook and the hook is supported with the some of the thumb and the fingers, and therefore the endoscope is stably held.

When the endoscope is used for a patient sitting and facing a medical doctor, for example, in a department of otorhinology, the endoscope may be used with a horizontal hold. That is, the proximal end portion of the operation portion is grasped with one hand from the proximal end side, and some of the thumb and the fingers of the one hand are placed on the bending operation portion. Then, while the endoscope held by the one hand is arranged sideway along a horizontal direction, the endoscope is appropriately moved and the bending operation portion is operated with some of the thumb and the fingers of the hand holding the endoscope.

The endoscopes disclosed in Jpn. Pat. Appln. KOKAI Publication Nos. 2002-282199 and 2005-237818 are not appropriate to the horizontal hold. For example, in the endoscope disclosed in Jpn. Pat. Appln. KOKAI Publication No. 2002-282199, the bending operation knob is not located within the widely directional length of the proximal end portion of the operation portion including the hook shaped part, with respect to the wide direction, and in the endoscope disclosed in Jpn. Pat. Appln. KOKAI Publication No. 2005-237818, the trackball is located on the more proximal end side than the proximal end portion of the operation portion including the hook. Therefore, in the state that the proximal end portion of the operation portion is enclosed with the palm of one hand from the proximal end side, some of the thumb and the fingers of the one hand are placed on the hook shaped part or the hook, and then the operation portion is securely grasped, and therefore the endoscope is stably held, it is difficult to operate the bending operation knob or the trackball with the others of the thumb and the fingers of the one hand. Moreover, for example, the hook shaped part and the hook protrudes in the one widely directional side. Therefore, it may be difficult to securely grasp the operation portion and therefore stably hold the endoscope as is mentioned above according to which of the right hand and the left hand the operation portion is grasped with and which of the front side and the back side the operation portion is grasped from with respect to a front-back direction wherein the front-back direction is orthogonal to the longitudinally axial direction and the wide direction.

Document US 2002/0184122 A1 discloses an endoscope having a long and narrow insertion part, and an operation part arranged at the base of the insertion part. The insertion part includes a bending portion and a tip. Further, the operation part is equipped with a curved knob that causes the bending portion to curve. A signal processing unit is detachably connected to the base of the operation part. The signal processing unit comprises a lamp which supplies light to a light guide in the endoscope, and a signal processing circuit for processing image signals of a CCD built into the tip of the endoscope. The signal processing unit further includes a transmitting circuit and an antenna for transmitting signals to the outside by radio.

US 2006/287575 discloses a endoscope with an antenna connected to one side of its grasping portion, arranged apart outwardly in the wide direction from the operation portion and configured to transmit a radio signal.

The present invention has been made in view of the above-mentioned problem, and is directed to provide the endoscope with a horizontal hold which inlcudes an antenna connected to one of its sides. The antenna extends so as to be inclined distally in the longitudinally axial direction of the endoscope and outwardly in the orthogonal direction to the axial direction.

The present invention provides an endoscope according to claim 1. Further aspects of the invention are explained in the depending claims.

In an aspect of the present invention, an endoscope configured to be carried with a hand is characterized by comprising: an insertion portion extending in a longitudinally axial direction and including a distal end portion and a proximal end portion; and an operation portion coaxially connected to the proximal end portion of the insertion portion and extending in the longitudinally axial direction, the insertion portion includes a bending portion configured to be actuated to be bent, the operation portion includes: an axially directional grasp portion extending in the longitudinally axial direction and configured to be grasped by an operator; a bending operation portion provided on a more proximal end side than the axially directional grasp portion and configured to operate the bending portion to be bent; and a widely directional grasp portion provided on a more proximal end side than the bending operation portion and configured to be grasped by an operator wherein at least a part of the bending operation portion is located within a widely directional length of the widely directional grasp portion with respect to a wide direction orthogonal to the longitudinally axial direction, and the widely directional grasp portion includes a grasp part protruding farther toward one widely directional side than the bending operation portion.

In the endoscope according to the present aspect, the grasp part of the widely directional grasp portion protrudes farther toward the one widely directional side than the bending operation portion and at least the part of the bending operation portion is located within the widely directional length of the widely directional grasp portion. Therefore, even in the state that the widely directional grasp portion is enclosed from the proximal end side with the palm of one hand, some of the thumb and the fingers of the one hand are placed on the grasp part, and then the operation portion is securely grasped, and therefore the endoscope is stably held, it is possible to easily operate the bending operation portion with the others of the thumb and the fingers of the one hand.

In a preferred aspect of the present invention, the endoscope configured to be carried with the hand is characterized in that the bending operation portion includes a rotational member rotatable about a rotational axis located along the wide direction, the rotational member includes a finger placement portion for operating on which at least one of a thumb and fingers of an operator are to be placed and configured to be rotated along the longitudinally axial direction, and at least a part of the finger placement portion for operating is located within a widely directional length of the widely directional grasp portion with respect to the wide direction.

In the endoscope according to the present aspect, it is possible to place at least one of the thumb and the fingers on the finger placement portion for operating of the rotational member and therefore easily operate the rotational member.

In a preferred aspect of the present invention, the endoscope configured to be carried with the hand is characterized by further comprising a rotational mechanism configured to rotate the widely directional grasp portion about a longitudinal axis with respect to the bending operation portion.

In the endoscope according to the present aspect, it is possible to arrange the widely directional grasp portion so as to facilitate operation for the bending operation portion by rotating the widely directional grasp portion with respect to the bending operation portion.

In an aspect of the present invention, an endoscope configured to be carried with a hand is characterized by comprising: an insertion portion extending in a longitudinally axial direction and including a distal end portion and a proximal end portion; an operation portion coaxially connected to the proximal end portion of the insertion portion and extending in the longitudinally axial direction, the operation portion includes: an operation portion body connected to the proximal end portion of the insertion portion; and an axially directional grasp portion provided in the operation portion body, extending in the longitudinally axial direction and configured to be grasped by an operator; and a widely directional grasp portion provided on a more proximal end side than the axially directional grasp portion and configured to be grasped by an operator, and the widely directional grasp portion includes: one side grasp part protruding farther toward one widely directional side than the operation portion body with respect to a wide direction orthogonal to the longitudinally axial direction; and other side grasp part protruding farther toward other widely directional side than the operation portion body.

In the endoscope according to the present aspect, the one side grasp part and the other side grasp part of the widely directional grasp portion protrudes farther toward the one and the other widely directional side than the operation portion body, respectively. Therefore, it is possible to stably hold the endoscope by enclosing the widely directional grasp portion from the proximal end side with the palm of one hand, and then placing some of the thumb and the fingers of the one hand on at least one grasp part, and therefore securely grasping the operation portion, whichever with the left hand and with the right hand and whichever from a front side and from a back side with respect to a front-back direction orthogonal to the longitudinally axial direction and the wide direction.

In a preferred aspect of the present invention, the endoscope configured to be carried with the hand is characterized in that at least one of the one side grasp part and the other side grasp part includes a finger placement portion for grasping formed on a longitudinally axially distal end side portion of the at least one of the grasp part and on which at least one of a thumb and fingers of an operator are to be put.

In the endoscope according to the present aspect, it is possible to securely grasp the widely directional grasp portion by placing at least one of the thumb and the fingers on the finger placement portion for grasping formed in the longitudinally axially distal end side portion of the grasp part and therefore grasping the widely directional grasp portion.

In a preferred aspect of the present invention, the endoscope configured to be carried with the hand is characterized in that the widely directional grasp portion has a smooth curved surface shape.

In the endoscope according to the present aspect, it is facilitated to grasp the widely directional grasp portion since the widely directional grasp portion has the smooth curved surface shape.

In a preferred aspect of the present invention, the endoscope configured to be carried with the hand is characterized by further comprising a rotational mechanism configured to rotate the widely directional grasp portion about a longitudinal axis with respect to the operation portion body.

In the endoscope according to the present aspect, it is possible to arrange the widely directional grasp portion so as to facilitate the grasping for the widely directional grasp portion by rotating the widely directional grasp portion with respect to the operation portion body.

The invention can be more fully understood from the following detailed description when taken in conjunction with the accompanying drawings, in which:
FIG. 1 is a schematic view showing an endoscope system according to an embodiment of the present invention;
FIG. 2 is a perspective view showing an endoscope according to the embodiment of the present invention;
FIG. 3 is a back side perspective view showing an operation portion according to the embodiment of the present invention;
FIG. 4 is a longitudinal sectional view showing the operation portion according to the embodiment of the present invention;
FIG. 5 is a schematic view showing the endoscope according to the embodiment of the present invention;
FIG. 6 is a front view showing a widely directional grasp portion according to the embodiment of the present invention;
FIG. 7 is a perspective view showing the widely directional grasp portion according to the embodiment of the present invention;
FIG. 8 is a side view showing a vertical hold for the endoscope according to the embodiment of the present invention;
FIG. 9 is a back view showing the vertical hold for the endoscope according to the embodiment of the present invention;
FIG. 10 is a side view showing a horizontal hold for the endoscope with the left hand from a front side according to the embodiment of the present invention;
FIG. 11 is a front view showing the horizontal hold for the endoscope with the left hand from the front side according to the embodiment of the present invention;
FIG. 12 is a perspective view showing the horizontal hold for the endoscope with the left hand from the front side according to the embodiment of the present invention;
FIG. 13 is a side view showing another horizontal hold for the endoscope with the left hand from a front side according to the embodiment of the present invention;
FIG. 14 is a side view showing the horizontal hold for the endoscope with the left hand from a back side according to the embodiment of the present invention;
FIG. 15 is a front view showing the horizontal hold for the endoscope with the right hand from a front side according to the embodiment of the present invention;
FIG. 16 is a back view showing the horizontal hold for the endoscope with the right hand from a back side according to the embodiment of the present invention;
FIG. 17 is a sectional view showing an attachment mechanism and a radio unit according to the embodiment of the present invention;
FIG. 18 is an exploded perspective view showing the attachment mechanism according to the embodiment of the present invention;
FIG. 19 is a sectional view showing the attachment mechanism according to the embodiment of the present invention;
FIG. 20A is a perspective view showing a rotation of the radio unit in one direction according to the embodiment of the present invention;
FIG. 20B is a perspective view showing a rotation of the radio unit in the other direction according to the embodiment of the present invention;
FIG. 21 is a perspective view showing the internal configure of the radio unit according to the embodiment of the present invention;
FIG. 22 is a perspective view showing the radio unit in the state that a battery housing portion is opened according to the embodiment of the present invention;
FIG. 23 is a perspective view showing the radio unit in the state that the battery housing portion is closed according to the embodiment of the present invention;
FIG. 24 is a top view showing the radio unit according to the embodiment of the present invention;
FIG. 25 is a front view showing a processing apparatus according to the embodiment of the present invention;
FIG. 26 is a back view showing the processing apparatus according to the embodiment of the present invention;
FIG. 27 is a perspective view showing a wire communicating system according to the embodiment of the present invention;
FIG. 28 is a top view showing a wire cable according to the embodiment of the present invention; and
FIG. 29 is a perspective view showing a radio environment information setting system according to the embodiment of the present invention.

An embodiment of the present invention will hereinafter be described with reference to the drawings.

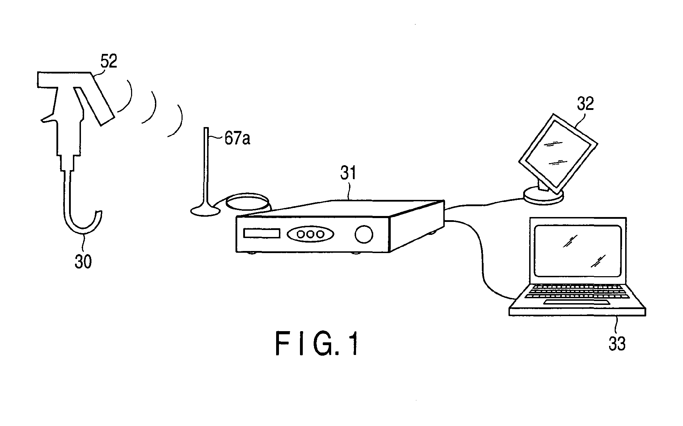

Referring to FIG. 1, a schematic configure of an endoscope system will be described.

An endoscope 30 of the endoscope system includes a radio unit 52. The radio unit 52 is configured to process an image pick-up signal of a subject to generate an image signal, and then convert the image signal into a radio signal and transmit the radio signal. A receiving antenna 67a is configured to receive the radio signal transmitted from the radio unit 52. The receiving antenna 67a is connected to a processing apparatus 31, and the processing apparatus 31 is configured to convert the radio signal into an image signal and then performs an image processing to the image signal. An image display apparatus 32 is connected to the processing apparatus 31 and configured to display an image on the basis of the image signal. A computer 33 is also connected to the processing apparatus 31 and is configured to perform store, process and the like of the image signal.

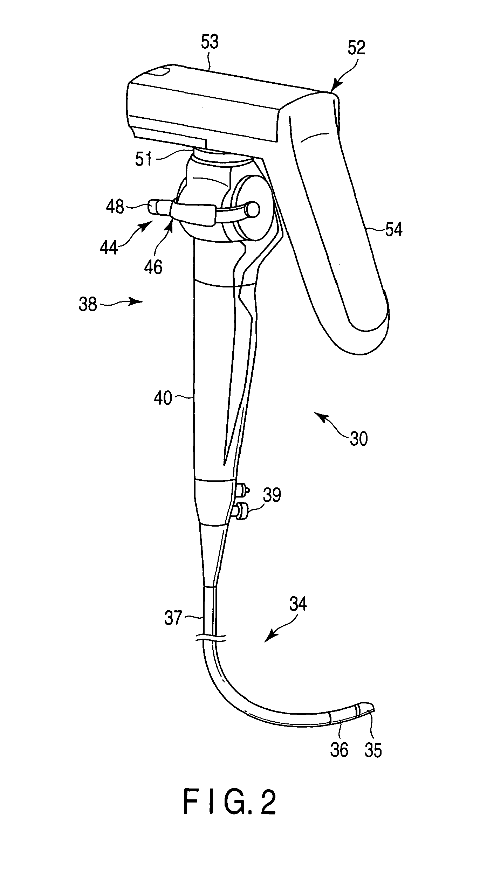

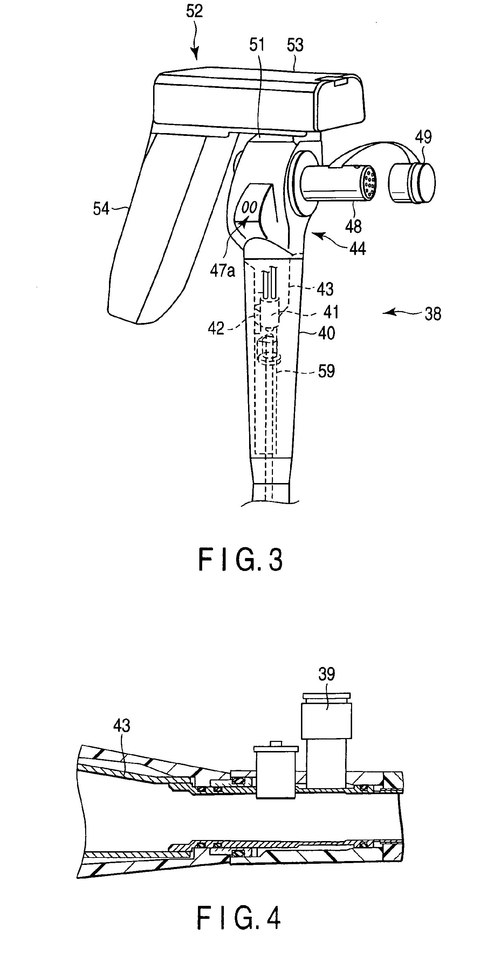

Referring to FIGS. 2 to 5, a schematic configure of the endoscope 30 will be described.

Referring to FIGS. 2 to 4, the endoscope 30 includes an insertion portion 34 extending in a longitudinally axial direction and configured to be inserted into the interior of the body. In the insertion portion 34, a distal end rigid portion 35, a bending portion 36 configured to be actuated to be bent, a long and flexible insertion tube portion 37 are provided from a distal end side to a proximal end side. An operation portion 38 is coaxially coupled to the proximal end portion of the insertion portion 34, extends in the longitudinally axial direction, and configured to be held and operated by an operator. Here, in the operation portion 38, a direction orthogonal to the longitudinally axial direction is referred to as a wide direction, and a direction orthogonal to the longitudinally axial direction and the wide direction is referred to as a front-back direction. A vent connecter 39 protrudes from the distal end portion of the operation portion 38 and fluidly communicates with the interior of the endoscope 30. In a water-tight test for the endoscope 30, an adapter of a blowing apparatus is connected to the vent connecter 39 and pressurized air is supplied to the interior of the endoscope 30 through the vent connecter 39. An axially directional grasp portion 40 is formed in the distal end side portion of the operation portion 38, extends in the longitudinally axial direction, and is configured to be grasped by an operator. A light source unit 41 is built in the axially directional grasp portion 40, includes a light-emitting diode, for example, and is configured to generate illumination light. The light source unit 41 is coupled to an inner frame 43 through a light source attachment portion 42. The inner frame 43 is coupled to the above vent connecter 39. The vent connecter 39 is a metal member exposed to the exterior of the endoscope 30, and therefore, heat generated in the light source unit 41 is to be radiated from the vent connecter 39 through the inner frame 43, thereby improving a heat radiation performance in the axially directional grasp portion 40. An operation lever portion 44 is formed on the proximal end portion of the operation portion 38. A bending operation lever 46 as a rotational member is provided on the front side of the operation lever portion 44 and is configured to operate the bending portion 36 to be bent. An operation lever portion switch portion 47a is provided on the back side of the operation lever portion 44 and is configured to operate the endoscope 30 and perform setting for the endscope 30, and remotely operate the processing apparatus 31. An endoscope connecter 48 for using the endoscope 30 with wire communication protrudes in the wide direction from the side surface of the operation lever portion 44. A cap 49 is connected to the endoscope connecter 48 and is configure to be mounted to the endoscope connecter 48 in order to keep the interior of the endoscope 30 water-tight in using the endoscope 30 with radio communication, sterilization, and the like. The L-shaped radio unit 52 is coupled to the proximal end portion of the operation lever portion 44 through an attachment mechanism 51. The radio unit 52 is formed of a widely directional grasp portion 53 located on the proximal end side with respect to the operation lever portion 44 and an antenna portion 54 coupled to the widely directional end portion of the widely directional grasp portion 53.

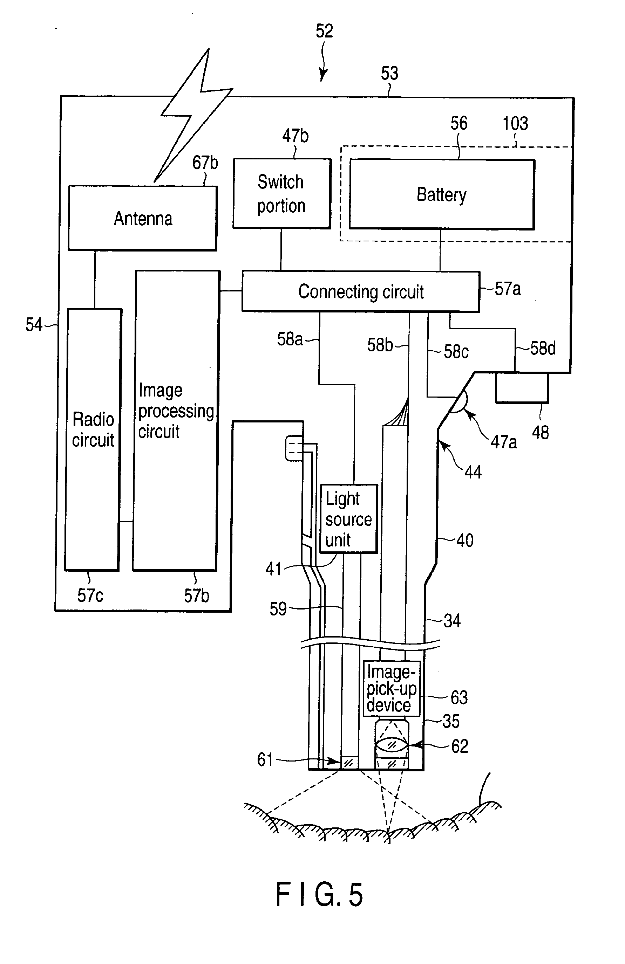

Referring to FIG. 5, a battery 56 functioning as a power source for the endoscope 30 is housed in the widely directional grasp portion 53 of the radio unit 52. The battery 56 is connected to a connecting circuit 57a in the widely directional grasp portion 53, and a power cable 58a extends from the connecting circuit 57a to the light source unit 41 in the axially directional grasp portion 40. Electric power is to be supplied to the light source unit 41 from the battery 56 through the connecting circuit 57a and the power source cable 58a, and illumination light is to be generated in the light source unit 41. The light source unit 41 is connected to an illumination optical system 61 built in the distal end rigid portion 35 through a light guide 59. Illumination light generated in the light source unit 41 is to be supplied to the illumination optical system 61 through the light guide 59, and the illumination light is to be emitted to the subject from the illumination optical system 61. The subject is to be formed into an image by an objective optical system 62 built in the distal end rigid portion 35, and the image is to be picked up by the image pick-up unit 63. The image pick-up unit 63 is connected to the connecting circuit 57a in the widely directional grasp portion 53 through an image pick-up cable 58b. An image pick-up signal generated in the image pick-up unit 63 is to be output to the connecting circuit 57a through the image pick-up cable 58b, the image pick-up signal is to be output to the image processing circuit 57b in the antenna portion 54 from the connecting circuit 57a, and the image pick-up signal is to be processed into an image signal in the image processing circuit 57b. The image signal is to be output to the radio circuit 57c in the antenna portion 54 from the image processing circuit 57b, and the image signal is to be converted into a radio signal in the radio circuit 57c. The image signal is to be output to the transmission antenna 67b from the radio circuit 57c, and the image signal is to be transmitted from the transmission antenna 67b. in addition, the operation lever portion switch portion 47a and the endoscope connecter 48 of the operation lever portion 44 are connected to the connecting circuit 57a through a switch wiring cable 58c and a connecter wiring cable 58d, respectively. Furthermore, a radio unit switch portion 47b is provided in the widely directional grasp portion 53 of the radio unit 52 and is configured to operate the endoscope 30 and perform setting for the endoscope 30, and remotely operate the processing apparatus 31, and the radio unit switch portion 47b is connected to the connecting circuit 57a.

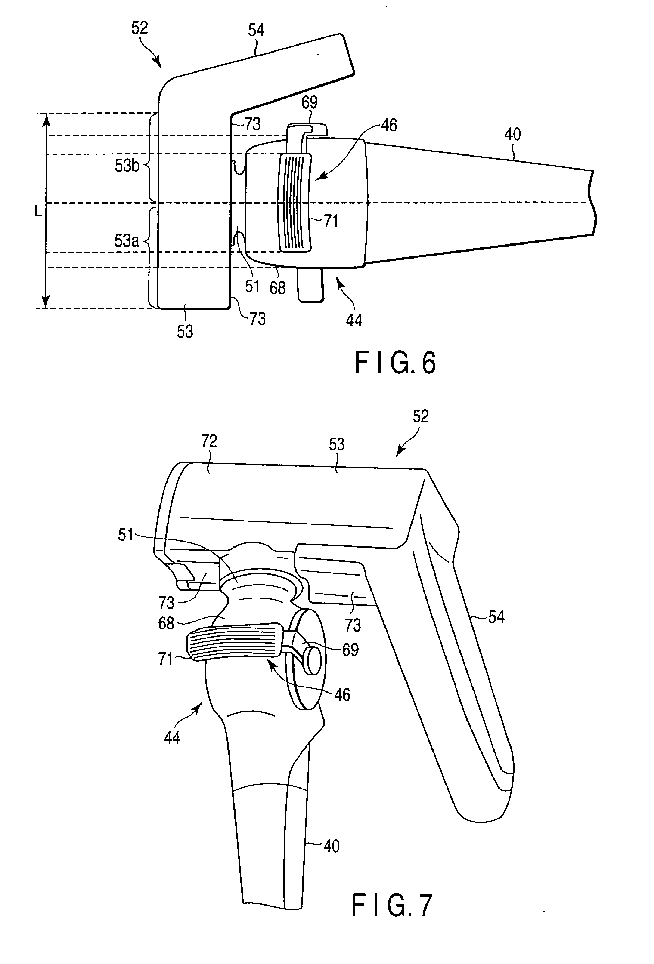

Referring to FIGS. 6 and 7, a grasp and operation mechanism in the endoscope 30 will be described.

The axially directional grasp portion 40 extends in the longitudinally axial direction in the distal end side portion of the operation portion 38.

An operation lever portion body 68 is located on a more proximal end side than the axially directional grasp portion 40, and the bending operation lever 46 is provided in the operation lever portion body 68. The bending operation lever 46 is formed of a rotational supporting portion 69 at the base side thereof and a finger placement portion for operating 71 at the terminal side thereof. That is, the base end portion of the rotational supporting portion 69 is rotatably attached to the widely directional side surface of the operation lever portion body 68, and the rotational supporting portion 69 extends from the back side to the front side and is rotatable about a rotational axis parallel with the wide direction. The base end portion of the finger placement portion for operating 71 is coupled to the terminal end portion of the rotational supporting portion 69. The finger placement portion for operating 71 is located on the front side of the operation lever portion body 68, extends in the wide direction, and is rotatable together with the rotational supporting portion 69 along the longitudinally axial direction.

The widely directional grasp portion 53 of the radio unit 52 is located on the more proximal end side than the operation lever portion body 68 through the attachment mechanism 51. At least a part of the bending operation lever 46 is located within the widely directional length of the widely directional grasp portion 53 with respect to the wide direction. In the present embodiment, the whole finger placement portion for operating 71 of the bending operation lever 46 is located within the widely directional length L of the widely directional grasp portion 53. One widely directional side part of the widely directional grasp portion 53 forms the one side grasp part 53a, and the other widely directional side part thereof forms the other side grasp part 53b. The one side grasp part 53a protrudes farther toward the one widely directional side than the bending operation lever 46 and the operation lever portion body 68. On the other hand, the other side grasp part 53b protrudes farther toward the other widely directional side than the bending operation lever 46 and the operation lever portion body 68.

Furthermore, in the present embodiment, the widely directional grasp portion 53 has a flat rectangular parallelepiped shape orthogonal to the longitudinally axial direction and extending in the wide direction, and the proximal end side portion of the widely directional grasp portion 53 has a flat surface shape orthogonal to the longitudinally axial direction and extending in the wide direction. In the proximal end side portion of the widely directional grasp portion 53, a ridgeline portion extending in the wide direction has a round shape 72, and the whole proximal end side portion of the widely directional grasp portion 53 has a smooth curved surface shape. Moreover, finger placement portions for grasping 73 are formed on both sides of the attachment mechanism 51 in the distal end side portion of the one side grasp part 53a and the other side grasp part 53b, respectively, and the cross section of the finger placement portion for grasping 73 orthogonal to the wide direction has a concave curved shape.

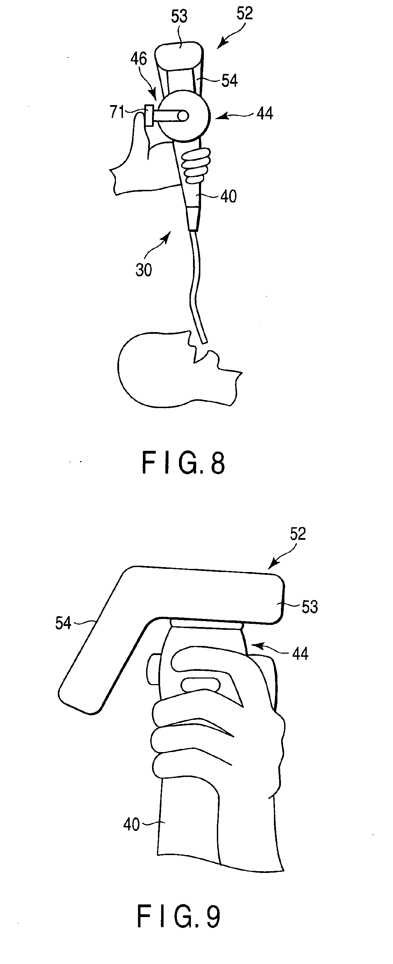

Referring to FIGS. 8 to 16, methods for grasping and operating the endoscope 30 will be described.

Referring to FIGS. 8 and 9, a method for grasping and operating the endoscope 30 with a vertical hold will be described.

When the endoscope 30 is used for a lying patient, the endoscope 30 is normally used with the vertical hold. That is, the axially directional grasp portion 40 is grasped so as to be enclosed with the palm of the left hand and the four fingers apart from the thumb thereof from the one widely directional side, and the thumb of the left hand is arranged on the front side with respect to the front-back direction and the proximal end side with respect to the longitudinally axial direction and is placed on the finger placement portion for operating 71 of the bending operation lever 46. Then, while the endoscope 30 held with the left hand is arranged upright along a vertical direction, the endoscope 30 is appropriately moved and the bending operation lever 46 is operated with the thumb of the left hand holding the endoscope 30.

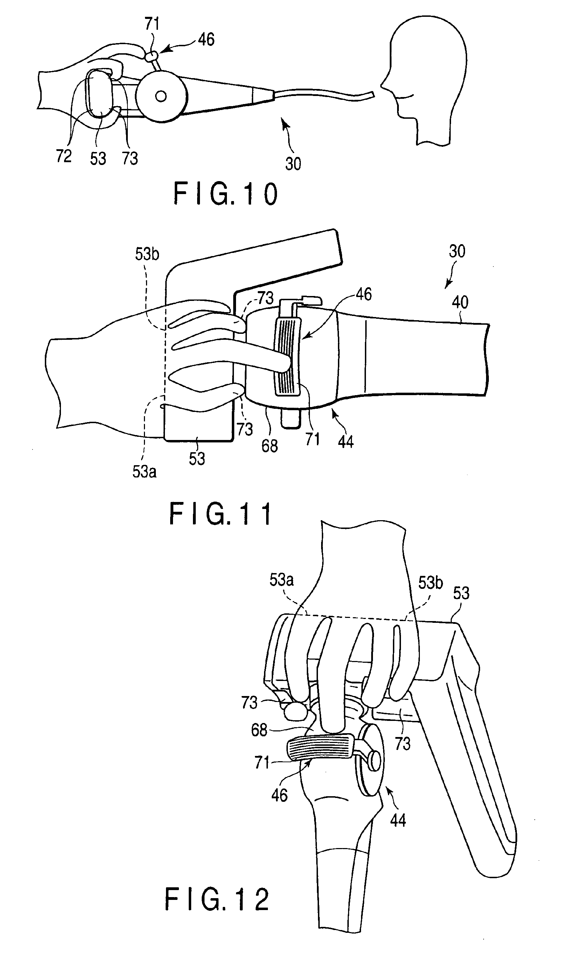

Referring to FIGS. 10 to 16, methods for grasping and operating the endoscope 30 with a horizontal hold will be described.

When the endoscope 30 is grasped and operated with the horizontal hold, there are various modes of hold according to each of operators and each of situations. Hereinafter, methods for grasping and operating will be described by example according to which of the right hand and the left hand the endoscope is grasped and operated with and which of the front side and the back side the endoscope 30 is grasped and operated from.

Referring to FIGS. 10 to 12, a horizontal hold with the left hand from the front side will be described.

When the endoscope 30 is used for a patient sitting and facing a medical doctor, for example, in a department of otorhinology, the endoscope 30 may be used with the horizontal hold. That is, the proximal end side portion of the widely directional grasp portion 53 is enclosed with the palm of the left hand, the first finger of the left hand is wound around the one side grasp part 53a from the front side and the tip of the first finger is placed on the finger placement portion for grasping 73, the thumb is wound around the one side grasp part 53a from the back side, the third finger and the fourth finger are wound around the other side grasp part 53b from the front side and the tip of the third finger is placed on the finger placement portion for grasping 73, and also the second finger is placed on the finger placement portion for operating 71 of the bending operation lever 46 from the front side, and therefore, the widely directional grasp portion 53 is grasped so as to be enclosed with the left hand. As a result, the operation portion 38 is securely grasped and therefore the endoscope 30 is stably held, and also the bending operation lever 46 can be easily operated in the state where the endoscope 30 is stably held. In particular, since the widely directional grasp portion 53 has a flat rectangular parallelepiped shape orthogonal to the longitudinally axial direction and extending in the wide direction, and the proximal end side portion of the widely directional grasp portion 53 has the flat surface shape orthogonal to the longitudinally axial direction and extending in the wide direction, it is facilitated to grasp the widely directional grasp portion 53 with the one hand from the proximal end side. Furthermore, since the ridgeline portion extending in the wide direction in the proximal end side portion of the widely directional grasp portion 53 has the round shape 72 and the whole proximal end side portion of the widely directional grasp portion 53 has the smooth curved surface shape, it is facilitated to grasp the widely directional grasp portion 53 with the palm of the hand. Next, while the endoscope 30 held with the left hand is arranged sideway along a horizontal direction such that the front side thereof turns vertically upward, the endoscope 30 is appropriately moved, and also the bending operation lever 46 is operated with the second finger of the left hand holding the endoscope 30.

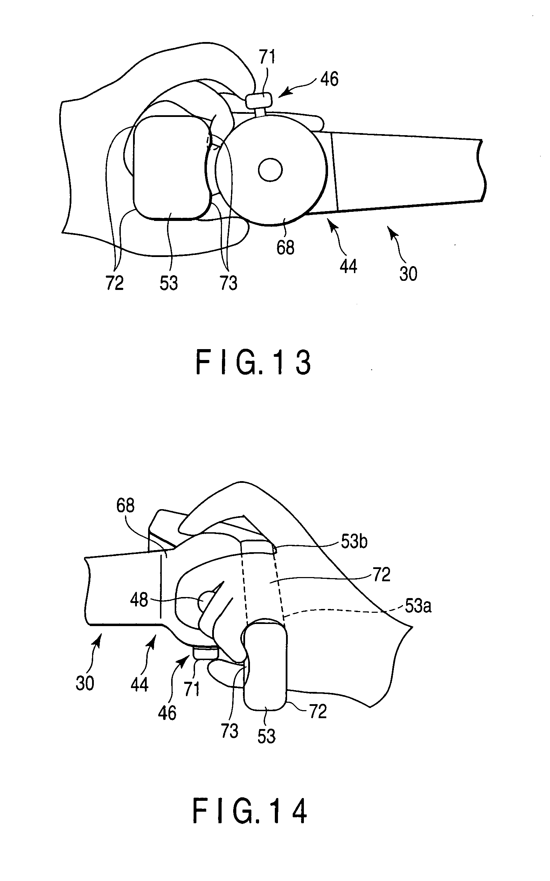

Referring to FIG. 13, another horizontal hold with the left hand from the front side will be described.

The proximal end side portion of the widely directional grasp portion 53 is enclosed with the palm of the left hand, the thumb of the left hand is wound around the one side portion of the grasp part 53a from the back side and the tip of the thumb is placed on the back surface of the operation lever portion body 68, the second finger and the third finger are wound around the other side grasp part 53b from the front side and the tips of the second finger and the third finger are placed on the finger placement portion for grasping 73, and also the first finger is placed on the finger placement portion for operating 71 of the bending operation lever 46 from the front side, and therefore, the widely directional grasp portion 53 is grasped so as to be enclosed with the left hand. Next, the endoscope 30 is arranged sideway along the horizontal direction such that the front side thereof turns vertically upward.

Referring to FIG. 14, a horizontal hold with the left hand from the back side will be described.

The proximal end side portion of the widely directional grasp portion 53 is enclosed with the palm of the left hand, the first finger of the left hand is wound around the other side grasp part 53 from the back side and the tip of the first finger is placed on the back surface of the operation lever portion body 68, the second finger, the third finger and the fourth finger are wound around the one side grasp part 53a from the back side, the second finger is placed on the endoscope connecter 48 in the present embodiment, the tips of the third finger and the fourth finger are placed on the finger placement portion for grasping 73, and also the thumb is placed on the finger placement portion for operating 71 of the bending operation lever 46 from the front side, and therefore, the widely directional grasp portion 53 is grasped so as to be enclosed with the left hand. Next, the endoscope 30 is arranged sideway along the horizontal direction such that the back side thereof turns vertically upward.

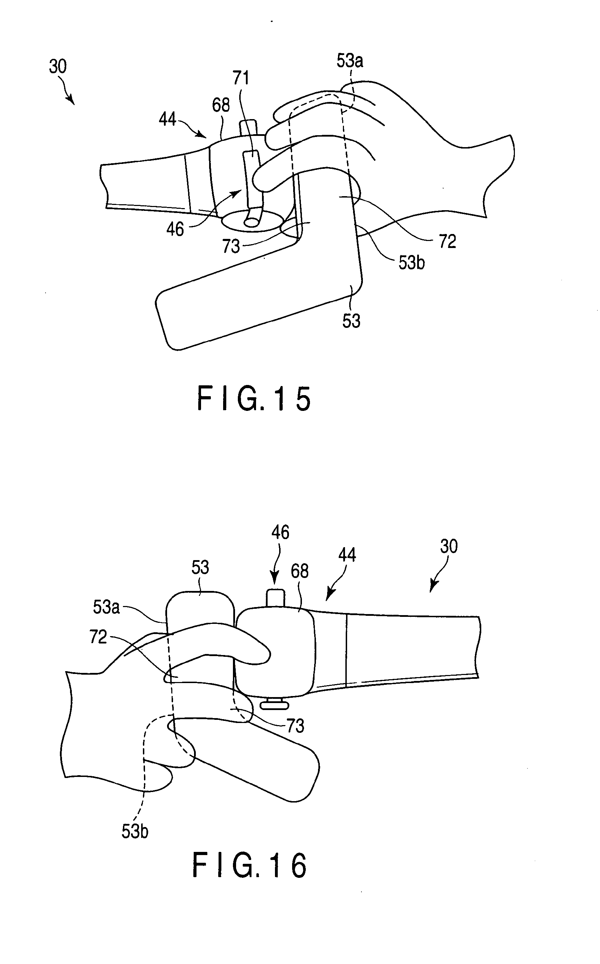

Referring to FIG. 15, a horizontal hold with the right hand from the front side will be described.

The proximal end side portion of the widely directional grasp portion 53 is enclosed with the palm of the right hand, the thumb of the right hand is wound around the other side grasp part 53b from the back side and the tip of the thumb is placed on the back surface of the operation lever portion body 68, the second finger, the third finger and the fourth finger are wound around the one side grasp part 53a from the front side and the tips of the second finger and the third finger are placed on the finger placement portion for grasping 73, and also the first finger is placed on the finger placement portion for operating 71 of the bending operation lever 46 from the front side, and therefore, the widely directional grasp portion 53 is grasped so as to be enclosed with the right hand. Next, the endoscope 30 is arranged sideway along the horizontal direction such that the front side thereof turns vertically upward.

Referring to FIG. 16, a horizontal hold with the right hand from the back side will be described.

The proximal end side portion of the widely directional grasp portion 53 is enclosed with the palm of the right hand, the first finger of the right hand is wound around the one side grasp part 53a from the back side and the tip of the first finger is placed on the back surface of the operation lever portion body 68, the second finger is wound around the other side grasp part 53b from the back side and the tip of the second finger is placed on the finger placement portion for grasping 73, and also the thumb is placed on the finger placement portion for operating 71 of the bending operation lever 46 from the front side, and therefore the widely directional grasp portion 53 is grasped so as to be enclosed with the right hand. Next, the endoscope 30 is arranged sideway along the horizontal direction such that the back side thereof turns vertically upward.

Here, a dimension such as a width or a thickness of the widely directional grasp portion 53 is set with respect to various ways of grasping such that the bending operation lever 46 can be easily operated while stably holding the endoscope 30. For example, the distance between the proximal end surface of the widely directional grasp portion 53 and the finger placement portion for operating 71 at the neutral position is set at 60 to 70 mm.

It is noted that in this application, the terms "the vertical hold" or "the horizontal hold" are used for referring to the relationship between the endoscope 30 and the hand holding the endoscope 30 and do not necessarily mean that the endoscope 30 is arranged along the vertical direction or the horizontal direction orthogonal to the vertical direction.

As is mentioned above, in the present embodiment, the grasp parts 53a and 53b of the widely directional grasp portion 53 protrudes farther in the wide direction than the finger placement portion for operating 71 of the bending operation lever 46 and the whole finger placement portion for operating 71 is located within the widely directional length of the widely directional grasp portion 53. Therefore, even in the state that the widely directional grasp portion 53 is enclosed with the palm of one hand from the proximal end side and the thumb and the fingers of the one hand is placed on the grasp part 53a, 53b, and then the operation portion 38 is securely grasped, and therefore the endoscope 30 is stably held, it is possible to easily operate the bending operation lever 46 with some of the thumb and the fingers of the one hand. It is noted that the similar effect is obtained when using the widely directional grasp portion 53 protruding toward only one side, although using the T-shaped widely directional grasp portion 53 protruding toward both widely directional sides with respect to the finger placement portion for operating 71 of the bending operation lever 46 in the present embodiment. For example, with respect to the wide direction, one side end of the one side grasp part 53a may be located slightly closer to the other side than the one side end of the finger placement portion for operating 71.

Furthermore, since the one side grasp part 53a and the other side grasp part 53b of the widely directional grasp portion 53 protrude farther toward the one widely directional side and the other widely directional side than the operation lever portion body 68, respectively, it is possible to stably holding the endoscope 30 by enclosing the widely directional grasp portion 53 with the palm of one hand from the proximal end side, and placing some of the thumb and the fingers of the one hand on the at least one grasp part 53a, 53b, and therefore securely grasping the operation portion 38, whichever the endscope 30 is held with the left hand and with the right hand and whichever the endoscope 30 is held from the front side and from the back side. In particular, it is possible to securely grasping the widely directional grasp portion 53 by placing some of the thumb and the fingers on the finger placement portion for grasping 73 formed in the proximal end side portion of the grasp part 53a, 53b, and therefore grasping the widely directional grasp portion 53. Furthermore, since the ridgeline portion extending in the wide direction in the proximal end side portion of the widely directional grasp portion 53 has the round shape 72 and the whole proximal end side portion of the widely directional grasp portion 53 has the smooth curved surface shape, it is facilitated to grasp the widely directional grasp portion 53 with the palm of the hand. It is noted that the widely directional grasp portion 53 may have any one of other various shapes as along as it includes a part protruding farther in the wide direction than the operation lever portion body 68, although the widely directional grasp portion 53 has the flat rectangular parallelepiped shape orthogonal to the longitudinally axial direction and extending in the wide direction in the present embodiment.

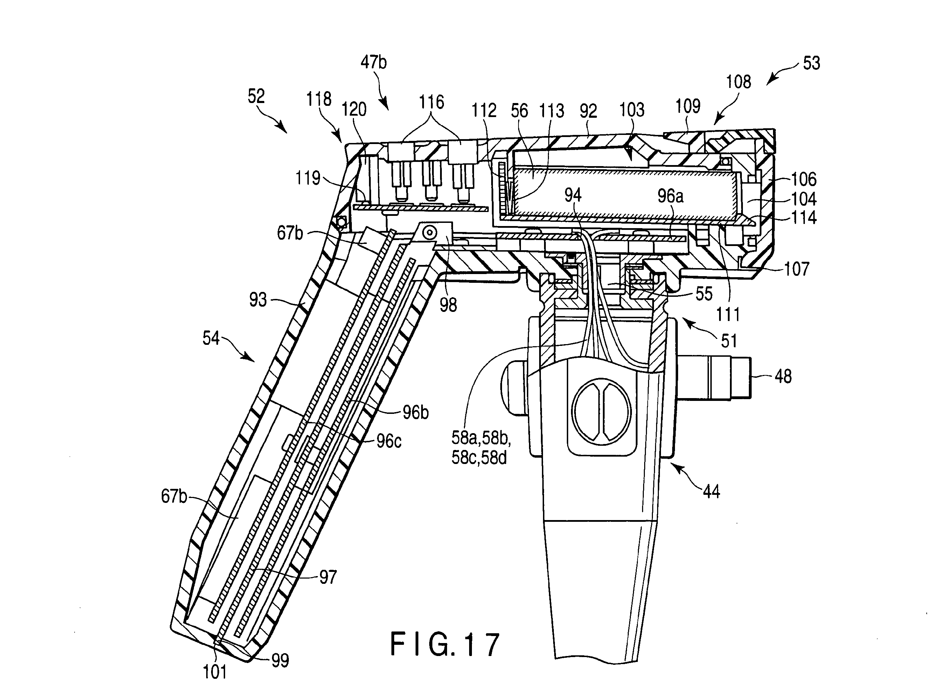

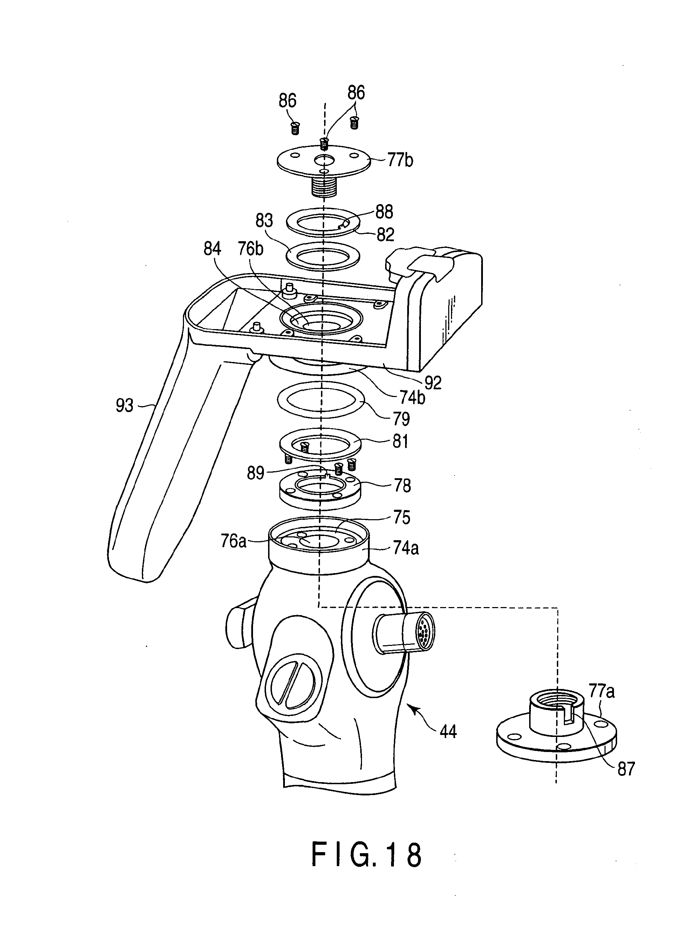

Referring to FIGS. 17 to 20B, the attachment mechanism 51 of the radio unit 52 as a rotational mechanism will be described.

The radio unit 52 is coupled to the operation lever portion 44 through the attachment mechanism 51 and is rotatable about a longitudinal axis within a certain range of a rotational angle with respect to the operation lever portion 44.

Referring to FIGS. 17 to 19, a first attachment portion 74a has a circularly cylindrical shape and extends in the longitudinally axial direction in the proximal end portion of the operation lever portion body 68. The first attachment portion 74a includes a partition 75 orthogonal to the longitudinally axial direction, and a first opening 76a having a circular shape about the longitudinal axis is formed in the partition 75. A stopper ring 78 has a thick circular ring shape and is coaxially located on and fixed to the longitudinally axially proximal end surface of the partition 75 of the first attachment portion 74a. A sliding ring 81 is coaxially located on the longitudinally axially proximal end surface of the stopper ring 78. On the other hand, a second attachment portion 74b has a circularly cylindrical shape and protrudes toward the longitudinally axially distal end from the longitudinally axially proximal end wall of the widely directional grasp portion 53. The second attachment portion 74b includes a bottom wall, and a second opening 76b has a circular shape about the longitudinal axis and is formed in the bottom wall. The second attachment portion 74b is coaxially fit onto the outside of the first attachment portion 74a of the operation lever portion body 68, and is rotatable about the longitudinal axis with respect to the first attachment portion 74a. The bottom wall of the second attachment portion 74b is locate on the end wall of the first attachment portion 74a and the sliding ring 81. It is noted that an O-ring 79 for keeping water-tight is interposed between the first attachment portion 74a and the second attachment portion 74b. Here, a first flange 77a is attached to the first attachment portion 74a from the inside of the operation lever portion body 68. That is, the first flange 77a is formed of a large outer diameter disk portion at the one end side portion thereof and a small outer diameter circularly cylindrical portion at the other end side portion thereof. The large outer diameter disk portion of the first flange 77a is coaxially located and fixed to the longitudinally axially distal end surface of the partition 75 of the first attachment portion 74a. The small outer diameter circularly cylindrical portion of the first flange 77a is inserted through the first opening 76a of the first attachment portion 74a, the inner space of the stopper ring 78, the inner space of the sliding ring 81 and the second opening 76b of the bottom wall of the second attachment portion 74b, and protrudes into the inside of the widely directional grasp portion 53. A brake pad 83 has a circular ring shape and is coaxially fit onto the outside of the small outer diameter circularly cylindrical portion of the first flange 77a within the widely directional grasp portion. The brake pad 83 is located on a pad receiving portion 84 formed on the inner surface of the proximal end wall of the widely directional grasp portion 53. A brake ring 82 has a circular ring shape and is coaxially located on the longitudinally axially proximal end surface of the brake pad 83, and the brake ring 82 is fit onto the outside of the small outer diameter circularly cylindrical portion of the first flange 77a. A second flange 77b is coaxially attached to the first flange 77a from the longitudinally axially proximal end side. That is, a internal thread is formed in the small outer diameter circularly cylindrical portion of the first flange 77a, the second flange 77b is formed of a large outer diameter disk portion at one end side portion thereof and a small outer diameter circularly cylindrical portion at the other end side portion thereof, and an external thread is formed in the small outer diameter circularly cylindrical portion. The small outer diameter circularly cylindrical portion of the second flange 77b is screwed into and attached to the small outer diameter circularly cylindrical portion of the first flange 77a.

Here, the brake ring 82 and the brake pad 83 are slidable in the longitudinally axial direction with respect to the first flange 77a. Threaded bores penetrate the large outer diameter disk portion of the second flange 77b in the longitudinally axial direction, and adjustment screws 86 are screwed into and attached to the threaded bores, respectively. The adjustment screw 86 protrudes toward the longitudinally axially distal end from the large outer diameter disk portion of the second flange 77b and presses the brake ring 82, and the brake ring 82 presses the brake pad 83 to the pad receiving portion 84. It is possible to adjust friction force to be applied between the brake pad 83 and the pad receiving portion 84 by adjusting an amount of protrusion of the adjustment screw 86, thereby adjusting press force to be applied between the brake pad 83 and the pad receiving portion 84. That is, it is possible to appropriately adjust an amount of rotational force needed to rotate the radio unit 52 with respect to the operation lever portion 44.

Moreover, a crevice 87 extends in the longitudinally axial direction in the small outer diameter circularly cylindrical portion of the first flange 77a, and a block portion 88 radially inwardly protrudes from the brake ring 82. The block portion 88 of the brake ring 82 is inserted into the crevice 87 of the first flange 77a. The brake ring 82 is slidable in the longitudinally axial direction with respect to the first flange 77a and rotation of the brake ring 82 about the longitudinal axis with respect to the first flange 77a is limited. Therefore, when the radio unit 52 is rotated with respect to the operation lever portion 44, the rotational force transmitted from the pad receiving portion 84 of the widely directional grasp portion 53 to the brake ring 82 through the brake pad 83 is intercepted in the brake ring 82, and no rotational force is transmitted from the brake ring 82 to the large outer diameter disk portion of the second flange 77b. Hence, it is prevented that the second flange 77b is rotated about the longitudinal axis with respect to the first flange 77a following rotation of the radio unit 52 with respect to the operation lever portion 44 and therefore the attachment by screwing of the second flange 77b with respect to the first flange 77a is loosened.

Furthermore, in the attachment mechanism 51, an attachment insertion bore 55 is formed of the inner space of the second flange 77b, and the interior of the operation lever portion 44 and the interior of the widely directional grasp portion 53 are communicated with each other through the attachment insertion bore 55. The power source cable 58a, the image pick-up cable 58b, the switch wiring cable 58c and the connecting wiring cable 58d respectively extended from the light source unit 41, image pick-up unit 63, the operation lever portion switch portion 47a and the endoscope connecter 48 are introduced into the widely directional grasp portion 53 from the interior of the operation lever portion body 68 through the attachment insertion bore 55, and are connected to the connecting circuit board 96a within the widely directional grasp portion 53. In this way, it is possible to perform wiring of various kinds of cable 58a, 58b, 58c and 58d together with each other with saving a space. Moreover, the various kinds of cable 58a, 58b, 58c and 58d may not be damaged in the attachment mechanism 51 even when the widely directional grasp portion 53 is rotated with respect to the operation lever portion 44.

Moreover, when the operation lever portion 44 is rotatable with respect to the radio unit 52 without limitation, various kinds of cable 58a, 58b, 58c and 58d can be damaged. In the present embodiment, a stopper portion 89 protrudes toward the longitudinally axially proximal end from the inner edge portion of the stopper ring 78. The stopper portion 89 is inserted through the inner space of the sliding ring 81, and inserted into a stopper groove 91 on the bottom wall of the second attachment portion 74b. The stopper groove 91 extends so as to have a circular arc shape about the longitudinal axis. When the stopper portion 89 is contact with the end wall of the stopper groove 91, the rotation of the radio unit 52 with respect to the operation lever portion 44 is limited.

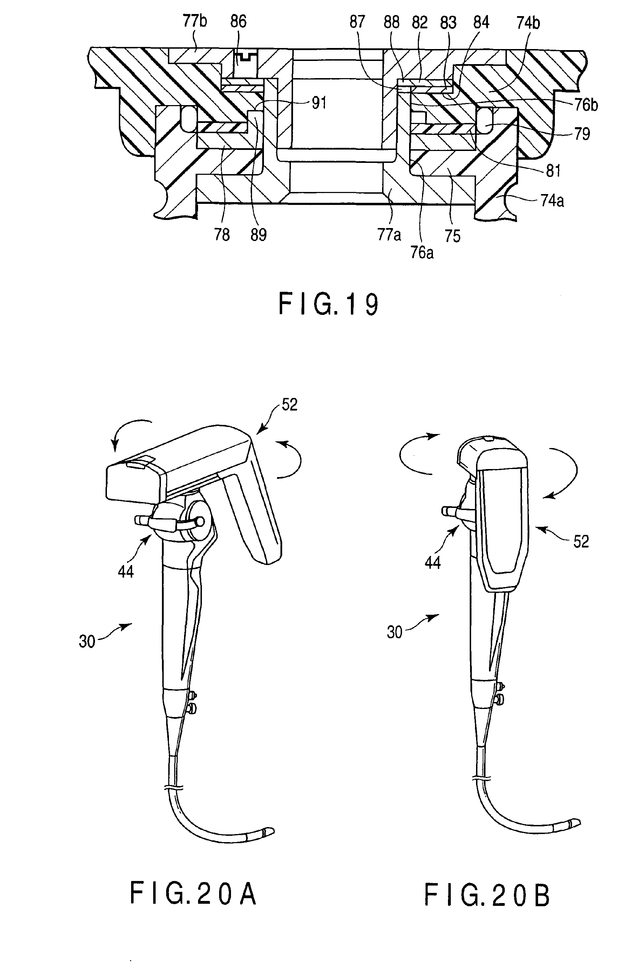

Furthermore, a range of a rotational angle of the radio unit 52 with respect to the operation lever portion 44 is appropriately set by setting a length and a position of the stopper groove 91 with respect to the peripheral direction appropriately. In the present embodiment, as is shown in FIG. 2, when the radio unit 52 is arranged at the neutral position, the widely directional grasp portion 53 extends in the wide direction, and the antenna portion 54 is arranged on the other widely directional side with respect to the bending operation lever 46. As is shown in FIGS. 20A and 20B, the radio unit 52 is rotatable in the right rotational direction and the left rotational direction by the same rotational angle, preferably by 90 degree with respect to the neutral position, when shown from the longitudinally axially proximal end side.

As is mentioned above, in the present embodiment, since the radio unit 52 is rotatable about the longitudinal axis with respect to the operation lever portion body 68 and the bending operation lever 46, it is possible to arrange the widely directional grasp portion 53 so as to facilitate grasping for the widely directional grasp portion 53 and operating for the bending operation lever 46 when grasping the widely directional grasp portion 53 and operating the bending operation lever 46 while holding the endoscope 30 with the horizontal hold. In particular, in the present embodiment, since the radio unit 52 is rotatable in the right rotational direction and the left rotational direction by the same rotational angle, it is possible to appropriately arrange the widely directional grasp portion 53 by rotating the radio unit 52 in the same manner whichever of the right hand and the left hand the horizontal hold is performed with and whichever of the front side and the back side the horizontal hold is performed from.

Moreover, it is possible to secure a good radio communication state by rotating the radio unit 52 with respect to the operation lever portion 44 so as to appropriately arrange the antenna portion 54 of the radio unit 52 according to various kinds of situation such as an environment in which the endoscope 30 is used and a posture of the endoscope 30 in use. Furthermore, in such a case where the endoscope 30 is carried with the vertical hold, it is possible to improve operability of the endoscope 30 by rotating the radio unit 52 with respect to the operation lever portion 44 such that the radio unit 52 is arranged not to hinder operation for the endoscope 30. In addition, in manufacturing for the endoscope 30, after the radio unit 52 is attached to the operation lever portion body 68 through the attachment mechanism 51, when the bending operation lever 46 is assembled to the operation lever portion body 68, it is possible to arrange the radio unit 52 so as to be shifted with respect to the base portion of the bending operation lever 46 such that it is facilitated to assemble the bending operation lever 46 to the operation lever portion body 68.

The radio unit 52 may be rotatable by 180 degree with respect to the neutral position, that is, the radio unit 52 may be reversible with respect to the operation lever portion 44. In this case, when the endoscope 30 is held with the horizontal hold, it is possible to arrange the radio unit 52 with respect to the hand in the same arrangement by appropriately reversing the radio unit 52 whichever of the front side and the back side the horizontal hold is performed from.

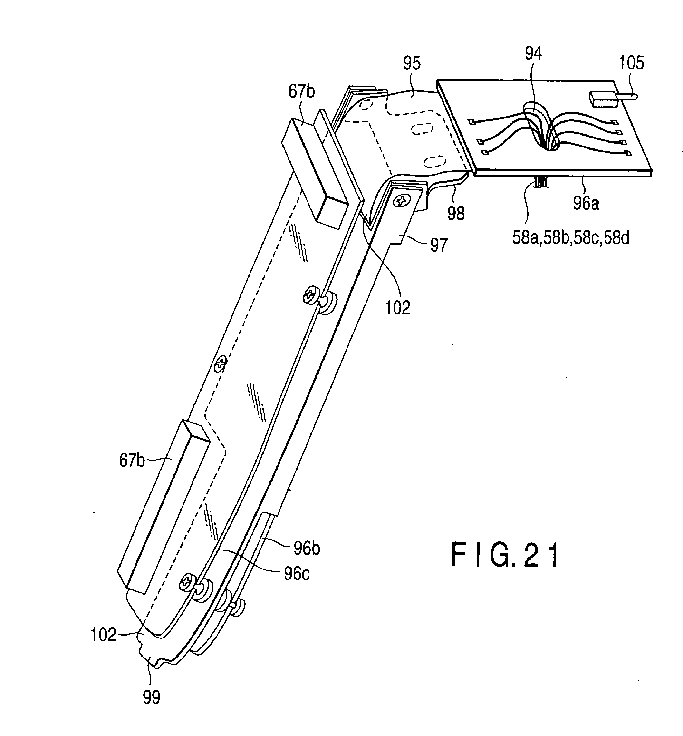

Referring to FIGS. 17, 18 and 21, the radio unit 52 will be described.

The radio unit 52 is formed of the widely directional grasp portion 53 and the antenna portion 54. A grasp portion housing 92 of the widely directional grasp portion 53 has a square cylindrical shape and extends in the wide direction. An antenna portion housing 93 of the antenna portion 54 has a square cylindrical shape extending so as to be inclined toward the longitudinally axially distal end and the other widely directional end from the other widely directionally end portion of the grasp portion housing 92 and has a pocket shape in which the terminal end portion thereof is closed.

In the grasp portion housing 92, the connecting circuit board 96a is provided on the proximal end side of the operation lever portion 44 and is orthogonally to the longitudinally axial direction. A board insertion bore 94 penetrates the connecting circuit board 96a along the longitudinal axis. Various kinds of cable 58a, 58b, 58c, 58d introduced into the widely directional grasp portion 53 from the operation lever portion 44 through the attachment insertion bore 55 of the attachment mechanism 51 are inserted through the board insertion bore 94 of the connecting circuit board 96a and are concentrically wired on the proximal end surface of the connecting circuit board 96a opposite to the operation lever portion 44. This facilitates wiring working and maintenance for the connecting circuit board 96a.

The connecting circuit board 96a is electrically connected to an image processing circuit board 96b within the antenna portion housing 93 through flexible board 95. Here, an attachment board 97 extends within the antenna portion housing 93 over the whole antenna portion 54. The image processing circuit board 96b is put on and fixed to the inside surface of the attachment board 97 at the side close to the operation lever portion 44. On the other hand, a radio circuit board 96c is put on and fixed to the outside surface of the attachment board 97 at the side opposite to the operation lever portion 44. The image processing circuit board 96b and the radio circuit board 96c are electrically connected to each other through an electric connecting part. The transmission antennas 67b are fixed to the outside surface of the radio circuit board 96c at the side opposite to the operation lever portion 44, and the transmission antenna 67b is located so as to face opposite to the operation lever portion 44. In this way, since the transmission antenna 67b is outwardly located at the most outer edge of the endoscope 30, a quality of radio communication is improved. The transmission antennas 67b are diversity antenna and are located orthogonally to each other such that directivities thereof are different from each other. Therefore, it is possible to transmit and receive a radio signal between the endoscope 30 and the processing apparatus 31 without uneven by rotating the radio unit 52 with respect to the operation lever portion 44 through the above rotation with one freedom in the attachment mechanism 51.

A coupling board 98 is coupled and fixed to one end portion of the attachment board 97, and the coupling board 98 is fixed to the inner surface of the longitudinally axially proximal end wall of the grasp portion housing 92 with vis. On the other hand, the other end portion of the attachment board 97 is contact with the inner surface of the terminal end portion of the antenna portion housing 93, a convex engaging portion 99 at the other end portion of the attachment board 97 is inserted into and engaged with the concave engaging receiving portion 101 at the inner surface of the terminal end portion of the antenna portion housing 93. In assembling the radio unit 52, the coupling board 98 is coupled to the attachment board 97 and each of the circuit boards 96b and 96c and the transmission antenna 67b is attached to the attachment board 97, and then the attachment board 97 is inserted into the antenna portion housing 93, the engaging portion 99 of the attachment board 97 is inserted into the engaging receiving portion 101 of the antenna portion housing 93, and the other end portion of the attachment board 97 is contact with the inner surface of the terminal end portion of the antenna portion housing 93. Next, the coupling board 98 is fixed to the inner surface of the longitudinally axially proximal end wall of the grasp portion housing 92 with vis, the attachment board 97 is urged toward the terminal end of the antenna portion 54, and therefore the attachment board 97 is held and fixed. In this way, an assembly performance and fixing strength of the attachment board 97 are improved.

Regarding the image processing circuit board 96b and the radio circuit board 96c, electrical devices to generate heat are mounted on a mount surface, and a mount surface side is located to face the attachment board 97 in order to efficiently transmit heat to the attachment board 97. Furthermore, a heat transmission member such as a heat transmission sheet and a gel sheet is interposed between the circuit board 96b, 96c and the attachment board 97. The attachment board 97 improves a heat radiation performance and prevents the electrical device from being damaged due to a rise in temperature of the circuit board 96b, 96c. The attachment board 97 also functions as a heat radiation board preventing a local rise in temperature in the antenna portion housing 93. For this reason, the attachment board 97 is made of metal having high heat conductivity. Heat transmitted to the attachment board 97 is transmitted to the grasp portion housing 92 through the coupling board 98 and then radiated to the endoscope body.

It is noted that communication quality may be deteriorated due to interception of the radio signal when a member containing metal is located to face the transmission antenna 67b. Therefore, a notch portion 102 is formed at each position facing each transmission antenna 67b in the attachment board 97 and the circuit board 96b.

As is mentioned above, the antenna portion 54 of the radio unit 52 is apart from the operation lever portion 44 toward the other widely directional side and extends to be inclined toward the longitudinally axially distal end and the other widely directional side. Therefore, it is facilitated to put the hand between the antenna portion 54 and the operation portion 38 when the endoscope 30 is held with the vertical hold. Moreover, since the antenna portion 54 is arranged radially outwardly apart from the hand grasping the operation portion 38, there is no touch with a part of the antenna portion 54 generating heat resulting in an uncomfortable feeling. On the other hand, when the endoscope 30 is held with the horizontal hold, since the endoscope 30 is arranged to be inclined and it is difficult to operate the bending operation lever 46 with the grasping hand when the antenna portion 54 is grasped from the proximal end side, it is prevented for an operator to grasp the antenna portion 54 by mistake. Therefore, it is prevented to touch a part of the antenna portion 54 generating heat resulting in an uncomfortable feeling, and also it is prevented to enclose the antenna portion 54 with hand resulting in deteriorating a quality of radio communication.

It is noted that, although the image processing circuit board 96b including the image processing circuit 57b, and the radio circuit board 96c including the radio circuit 57c are used in the present embodiment, any circuit configuration may be used as long as the whole two circuit board contains the image processing circuit 57b and the radio circuit 57c.

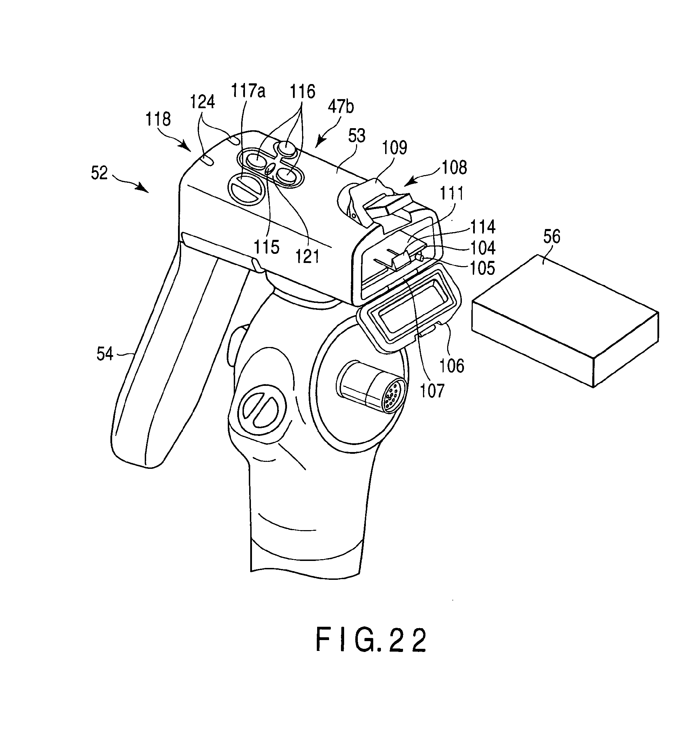

Referring to FIGS. 17, and 22 to 24, the battery housing portion 103 will be described.

The battery housing portion 103 is formed on the opposite side to the antenna portion 54 in the widely directional grasp portion 53.

That is, the grasp portion housing 92 of the widely directional grasp portion 53 has a square cylindrical shape, one widely directional end portion of the grasp portion housing 92 opens to form a battery housing opening 104 through which the battery 56 is to be attached to and detached from. The battery housing opening 104 is configured to be opened and closed by a battery cover 106. That is, the board shaped battery cover 106 includes one side portion extending in the front-back direction, and the one side portion is coupled to the longitudinally axially proximal end wall of the grasp portion housing 92 through a hinge 107 in the one widely directional end portion of the grasp portion housing 92. The battery cover 106 is rotatable about a rotational axis extending in the front-back direction, and is configured to be switched between a close position where the battery cover 106 is located on the longitudinally axially proximal end side and closes the battery housing opening 104, and an open position where the battery cover 106 is located on the longitudinally axially distal end side and opens the battery housing opening 104. A buckle mechanism 108 is provided in the longitudinally axially end wall of the grasp portion housing 92 in the one widely directional end portion of the grasp portion housing 92, and is configured to keep the battery cover 106 in the close position. The buckle mechanism 108 is configured to be switched between a hold state where the buckle mechanism 108 holds the battery cover 106 and a release state where the buckle mechanism 108 releases the battery cover 106 through switching operation to the buckle lever 109. In the hold state, the buckle mechanism 108 forms the same surface as the proximal end surface of the grasp portion housing 92 around the buckle mechanism 108 and the whole buckle mechanism 108 forms a smooth surface shape.

A supporting board 111 is provided within the grasp portion housing 92, faces to the longitudinally axially distal end wall of the grasp portion housing 92, is orthogonal to the longitudinally axial direction, and is configured to support the battery 56. Moreover, a dividing board 112 is provided within the grasp portion housing 92, opposes to the battery housing opening 104 and is orthogonal to the wide direction. The dividing board 112 limits displacement of an electrode 113 configured to be contact with an electric contact of the battery 56. The battery cover 106, the longitudinally axially end wall of the grasp portion housing 92, the supporting board 111, and the dividing board 112 forms a housing space configured to house the battery 56. In order to prevent deterioration in balance in holding the endoscope 30, the center of gravity of the radio unit 52 is preferably located on the longitudinal axis, and the center of gravity of the battery 56 housed in the housing space is located so as to be shifted toward the one widely directional side opposite to the antenna portion 54 with respect to the longitudinal axis in order to balance the battery 56 with the antenna portion 54.

An engaging claw 114 protrudes from the widely directional end portion of the supporting board 111 toward the longitudinally axially proximal end near the battery housing opening 104. The engaging claw 114 is configured to engage the battery 56 housed in the housing space and keep the battery 56 in a housing position. It is possible to easily release the engagement of the engaging claw 114 by pressing the engaging claw 114 toward the longitudinally axially distal end and smoothly take the battery 56 in and out. Therefore, in such a case where the battery cover 106 is opened by mistake in using the endoscope 30 or the battery 56 is taken out, the battery 56 is prevented from coming out and falling due to weight thereof. Moreover, it is facilitated to perform exchanging working for the battery 56.

A detecting switch 105 is provided in the one widely directional end portion of the grasp portion housing 92 and is configured to detect the opening and closing of the battery cover 106. When the battery cover 106 is opened by mistake in actuating the endoscope 30, it is indicated to an operator that the battery cover 106 is opened, for example, by displaying warning or stopping the image on the image display apparatus 32.

Referring to FIGS. 17, and 22 to 24, a radio unit switch portion 47b will be described.

The radio unit switch portion 47b is provided on the side of the widely directional grasp portion 53 close to the antenna portion 54.

That is, operation switches 116 are provided in the widely directional end portion of the widely directional grasp portion 53. The pressed portion of the operation switch 116 is located on the inside of the surface of the widely directional grasp portion 53 such that the pressed surface of the operation switch 116 is the substantially same surface as the surface of the widely directional grasp portion 53. Therefore, the operation switch 116 is prevented from being operated by an operator by mistake when the widely directional grasp portion 53 is grasped with the horizontal hold. Moreover, grooves are formed around the operation switches 116 so as to facilitate pressing operation to the operation switches 116. The grooves are coupled to each other, and the coupled groove 121 is connected to the back surface as the outer surface of the widely directional grasp portion 53 through a smooth curved surface. This improves a drain around the operation switches 116 and prevents a collection of dirty water and the like around the operation switches 116. Moreover, in the coupled groove 121, a protrusion 115 is formed between the operation switches 116 adjacent to each other as a division. This prevents the operation switches 116 adjacent to each other from being simultaneously operated by mistake.

The radio unit switch portion 47b includes the radio unit changeover switch 117a for selecting a radio channel out of radio channels. The setting for the radio channel is performed in order to prevent interference between radio communications when simultaneously using the radio endoscope systems. A dial switch is used as the radio unit changeover switch 117a, and therefore it is possible to reduce a circuit size, thereby realizing miniaturization and cost reduction in the radio unit 52, and also it is possible to keep the selected setting for the radio channel even when the power source supply is turned off.

Referring to FIGS. 17 and 22 to 24, indicating portions 118 of the radio unit 52 will be described.

The indicating portions 118 are provided in the end portion of the widely directional grasp portion 53 close to the antenna portion 54 and are configured to indicate remaining battery life, a communication state, and the like.

That is, an indicating lamp 119 such as LED is provided within the widely directional grasp portion 53. Light form the indicating lamp 119 is transmitted through a light transmitting board 120. The light transmitting board 120 is exposed to the outside over the proximal end surface and the widely directional end surface of the widely directional grasp portion 53 in the edge portion of the widely directional grasp portion 53, and forms a light emitting portion 124. Therefore, it is facilitated to visually confirm the light emitting portion 124 in either case where the endoscope 30 is carried with the vertical hold or the horizontal hold.

In the present embodiment, a pair of indicating lamp 119 is used. One indicating lamp 119 is configured to indicate the remaining battery life, and the other indicating lamp 119 is configured to indicate the communication state. For example, the one indicating lamp 119 is configured to emit green light when the remaining battery life is sufficient and to emit yellow light when the remaining battery life is small. The other indicating lamp 119 is configured to emit green light when the communication state is normal and to emit yellow light when the communication state is abnormal. Both indicating lamps 119 are configured to be turned off in a wire communication state described below. In addition, the indicating lamp 119 may be configured to emit various color lights in various patterns and to be turned on and off according to the state of the endoscope 30.

It is noted that the remaining battery life may be displayed in the image display apparatus 32 besides indicated in the indicating portion 118 of the endoscope 30. That is, remaining battery life information is to be transmitted from the endoscope 30 to the processing apparatus 31 through radio communication, and an indicator indicating the remaining battery life is to be displayed on the image display apparatus 32 by the processing apparatus 31. The remaining battery life information displayed on the image display apparatus 32 may be more detailed than the remaining battery life information indicated by the indicating portion 118 of the endoscope 30. For example, the remaining battery life may be indicated by the indicating portion 118 of the endoscope 30 by two steps of green and yellow and, in contrast, the remaining battery life may be indicated in the image display apparatus 32 by three steps that three symbols are used for expressing the remaining battery life and each of three symbols is deleted in order according to reduction in the remaining battery life.

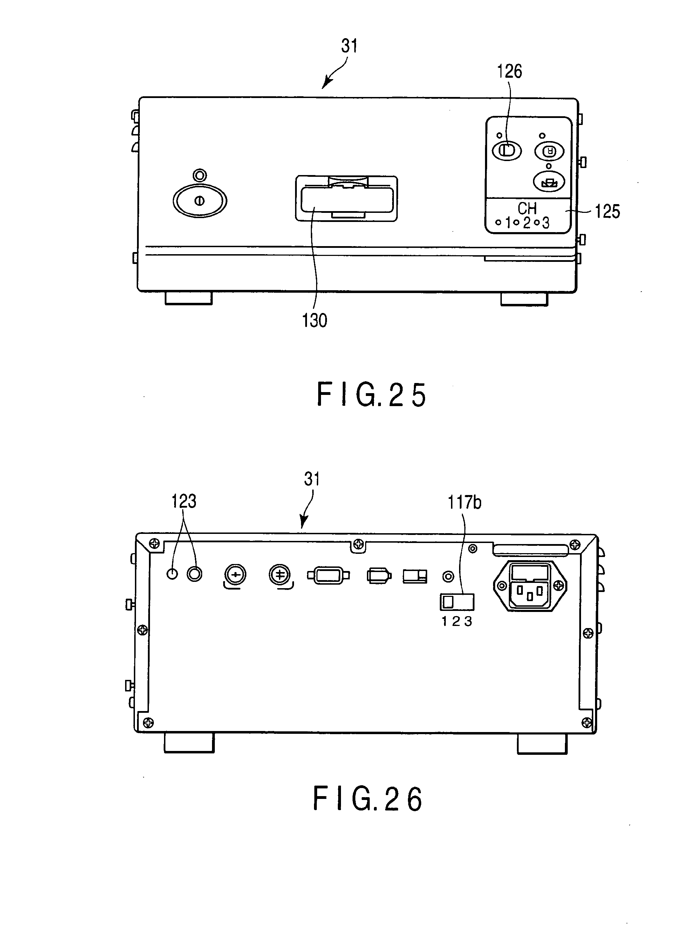

Referring to FIGS. 25 and 26, the processing apparatus 31 will be described.

Antenna connecters 123 is provided on the back surface of the processing apparatus 31, and each of receiving antennas 67a is connected to each of the antenna connecters 123. Therefore, since a radio signal is received continually using the receiving antenna 67a having a good receiving state out of the receiving antennas 67a, it is possible to prevent a quality of communication from being deteriorated.

In addition, a processing apparatus changeover switch 117b is provided in the back surface of the processing apparatus 31, and is configured to select a radio channel out of radio channels corresponding to radio channels of the radio unit 52 of the endoscope 30. A channel indicating portion 125 is provided on the front surface of the processing apparatus 31, and is configured to display the selected radio channel. Since radio communication is performed only between the endoscope 30 and the processing apparatus 31 in which the radio channels thereof are set at the same, interference between the radio communications is prevented.

Color-bar as test pattern may be displayed on the image display apparatus 32 by the processing apparatus 31 in order to check whether the processing apparatus 31 and the image display apparatus 32 properly function or not. In many wire endoscope systems, the color-bar is automatically displayed by the image display apparatus 32 when the image signal is not received by the processing apparatus 31. In the case where such a processing apparatus 31 is used in the radio endoscope system, when a radio communication state is deteriorated in using the endoscope 30, the color-bar may be suddenly displayed on the image display apparatus 32 so that a surgical operation is hampered. For this reason, in the endoscope system according to the present invention, the color-bar is not automatically displayed on the image display apparatus 32 when the image signal is not received. Instead of this, a color-bar switch 126 is provided on the front surface of the processing apparatus 31 and the color-bar is to be displayed according to operation to the color-bar switch 126. Therefore, it is possible to display the color-bar only when necessary.

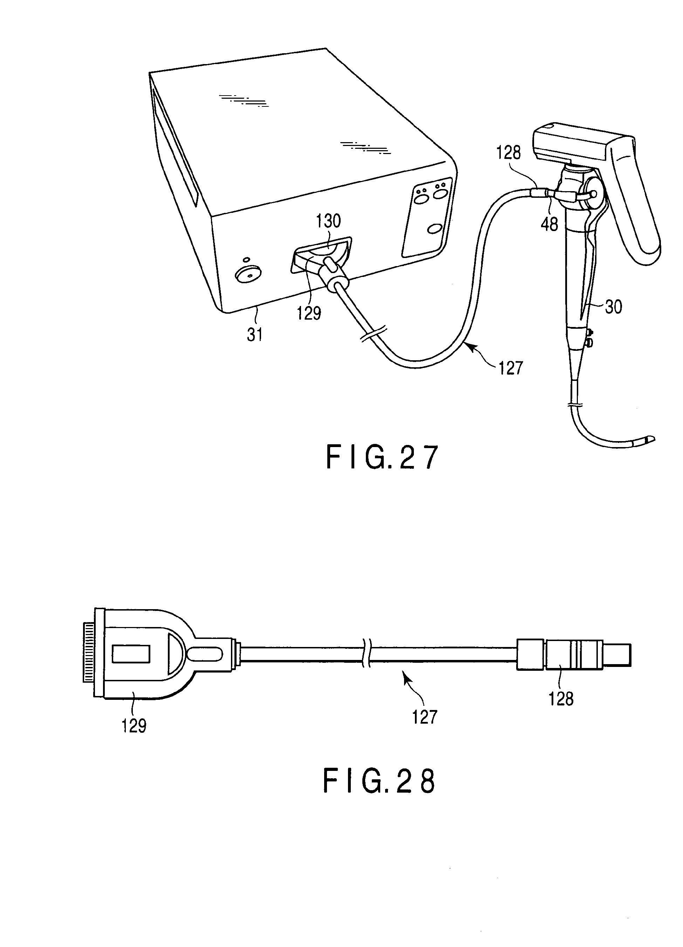

Referring to FIGS. 27 and 28, a wire communicating system will be described.

The endoscope system includes a wire cable 127. A cable connecter 128 is provided on one end portion of the wire cable 127 and is configured to be connected to the endoscope connecter 48 of the endoscope 30, and a plug 129 is provided on the other end portion of the wire cable 127 and is configured to connect a receptacle 130 of the processing apparatus 31.

In such a case where remaining battery life of the battery 56 equipped in the endoscope 30 become a little or a radio communication condition become worse, the endoscope system is switched from the radio communication to the wire communication. That is, the cable connecter 128 of the wire cable 127 is connected to the endoscope connecter 48 of the endoscope 30, and the plug 129 of the wire cable 127 is connected to the receptacle 130 of the processing apparatus 31. When the endoscope 30 and the processing apparatus 31 are connected to each other through the wire cable 127, each of the circuits is switched, electric power supply from the battery 56 equipped in the endoscope 30 is stopped, electric power is supplied from the processing apparatus 31 to the endoscope 30 through the wire cable 127. Moreover, a radio communication function in the endoscope 30 and the processing apparatus 31 is stopped, and a signal is transmitted and received through the wire cable 127 between the endoscope 30 and the processing apparatus 31. In this way, even in the case where the remaining battery life of the battery 56 equipped in the endoscope 30 become a little and the radio communication condition become worse, the endoscope system can stably function.

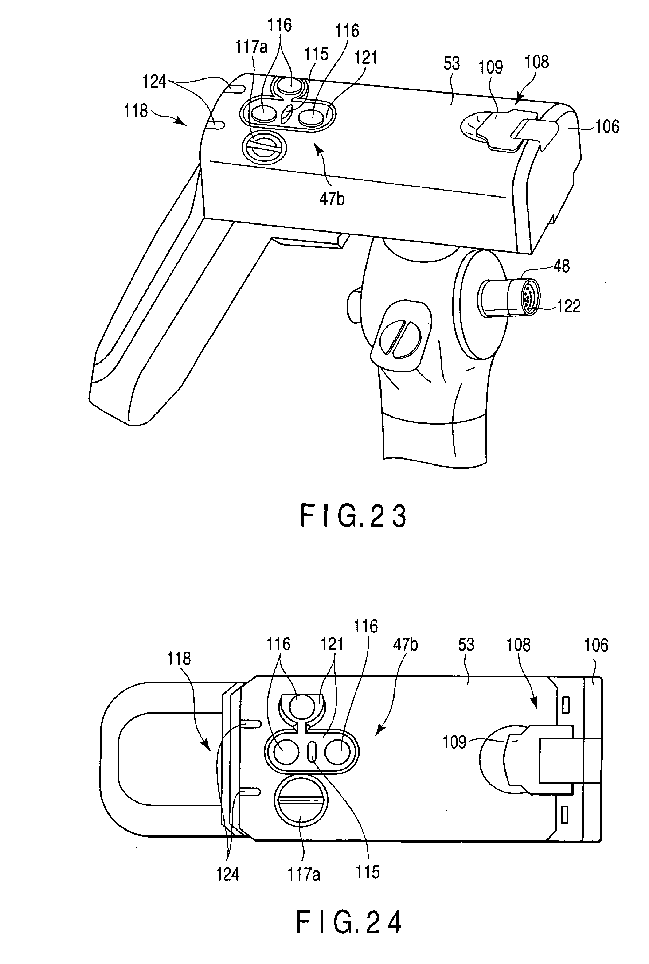

In addition, referring to FIG. 23, a hollow pin 122 is formed in the endoscope connecter 48. The hollow pin 122 is not used for wiring and a signal line of the connecter wiring cable 58d is not connected to the hollow pin 122 by soldering. The exterior and the interior of the endoscope 30 are fluidly communicated with each other through the hollow pin 122. In a water-tight test of the endoscope 30, an adapter of a blowing apparatus is connected to the vent connecter 39 of the endoscope 30, pressurized air is supplied into the interior of the endoscope 30, the endoscope 30 is sunk into the water, and it is confirmed whether there is a leak of air from the insertion portion 34, that is, a tear of the insertion portion 34 or not. In this time, when the cable connecter 128 of the wire cable 127 is connected to the endoscope connecter 48, it is possible to confirm water-tight in the connecting region wherein the endoscope connecter 48 and the cable connecter 128 are connected to each other since pressurized air is supplied into the connecting region from the interior of the endoscope 30 through the hollow pin 122. In addition, when the cap 49 is attached to the endoscope connecter 48, it is possible to confirm water-tight with the cap 49. It is noted that, in the case where the vent connecter 39 is not provided in the endoscope 30, an adapter of a blowing apparatus is connected to the endoscope connecter 48, pressurized air is supplied to the interior of the endoscope 30 through the hollow pin 122, and a water-tight test is performed.

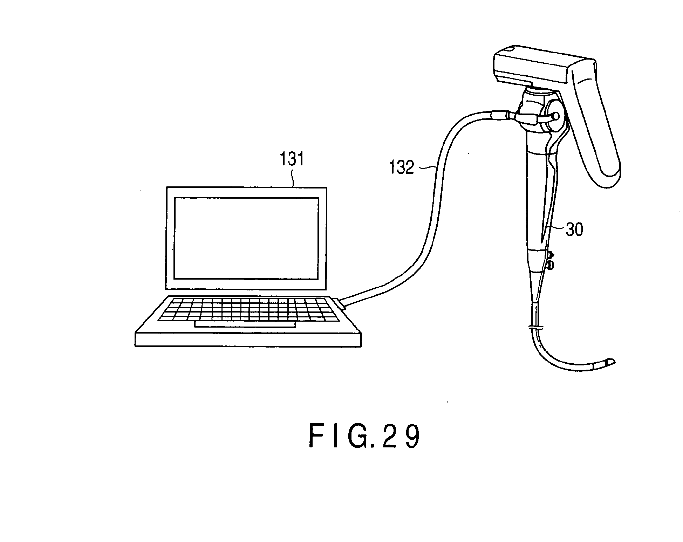

Referring to FIG. 29, a radio environment information setting system will be described.

Regarding radio instrument, there is an instrument wherein it is necessary to write in the instrument the radio environment setting information decided in each country such as radio LAN mode. When preparing for each supplied area kinds of model in which the radio environment setting information for each country is written, this brings about an increased complication of management and a rise in a price. Therefore, the radio environment setting information is usually written into the radio instrument for each supplied area before shipment. When writing the radio environment setting information in the endoscope only through radio communication, it is necessary to equip the endoscope with a writing circuit configured to write the radio environment setting information as well as a written circuit in which the radio environment setting information is to be written. This brings about an increased complication of circuits, an increased volume, and an increased price in the endoscope. In this reason, wire communication is preferable in writing the radio environment setting information in the endoscope. For example, the endoscope connecter 48 of the endoscope 30 and the writing apparatus 131 is connected to each other through the connecting cable 132, the radio environment setting information is written in the endoscope 30 through the connecting cable 132 by the writing apparatus 131.

Although the radio endoscope system is described in the above embodiment as an example, the present invention can be applied to various endoscope systems.

## Claims

1. An endoscope configured to be carried with a hand, comprising:
an insertion portion (34) extending in a longitudinally axial direction, including a distal end portion and a proximal end portion and configured to be inserted into a body; and
an operation portion (38) coaxially connected to the proximal end portion of the insertion portion (34), extending in the longitudinally axial direction and configured to be held and operated by an operator, and
wherein the insertion portion (34) includes a bending portion (36) configured to be bent, and
the operation portion (38) includes:
a first grasp portion (40) extending in the longitudinally axial direction and configured to be grasped by an operator;
a bending operation portion body (68) arranged on a more proximal end side in the longitudinally axial direction than the first grasp portion (40) and including a proximal end portion;
a bending operation lever (46) provided in the bending operation portion body (68), arranged on a more proximal end side in the longitudinally axial direction than the first grasp portion (40) configured to operate the bending portion (36) to be bent; and including a supporting portion (69) and a finger placement portion for operating (71), wherein the supporting portion (69) is configured to rotate about a rotational axis extending in a wide direction orthogonal to the longitudinally axial direction and the finger placement portion for operating (71) is coupled to the supporting portion (69), wherein the finger placement portion for operating (71) extends in the wide direction and is configured to move along the longitudinally axial direction by a rotation of the supporting portion (69), wherein at least one of a thumb and fingers of an operator can be placed on the finger placement portion for operating (71); and
a radio unit (52) connected to the proximal end portion of the bending operation portion body (68) and configured to perform a radio communication,
**characterized in that**
the radio unit (52) includes:
a second grasp portion (53) arranged on a more proximal end side in the longitudinally axial direction than the bending operation portion body (68), extending in the wide direction, configured to be grasped by an operator and including a first and a second grasp part (53a, 53b), wherein the first grasp part (53a, 53b) is provided on one side in the wide direction, of the second grasp portion (53), and protrudes outwardly in the wide direction farther than the bending operation lever (46) and the bending operation portion body (68), the second grasp part (53b, 53a) is provided on other side in the wide direction opposite to the one side, of the second grasp portion (53), and protrudes outwardly in the wide direction farther than the bending operation lever (46) and the bending operation portion body (68), and the whole finger placement portion for operating (71) is located within the length of the second grasp portion (53), with respect to the wide direction; and
an antenna (54) connected to one end in the wide direction, of the second grasp portion (53), extending so as to be inclined distally in the longitudinally axial direction and outwardly in the wide direction, arranged apart outwardly in the wide direction from the operation portion (38) and configured to transmit a radio signal.

2. The endoscope configured to be carried with the hand according to claim 1, wherein
the grasping part (53a, 53b) includes a finger placement portion for grasping (73) provided on a distal side in the longitudinally axially direction, of the grasping part (53a, 53b), and on which at least one of a thumb and fingers of an operator are to be put.

3. The endoscope configured to be carried with the hand according to claim 1, wherein
the second grasp portion (53) has a smooth curved surface shape (72).

4. The endoscope configured to be carried with the hand according to claim 1, further comprising
a rotational mechanism (51) configured to rotate the radio unit (52) about a longitudinal axis with respect to the bending operation portion body (68).

## Patentansprüche

1. Endoskop, das dazu eingerichtet ist, mit einer Hand gehalten zu werden und das umfasst:
einen Einführabschnitt (34), der sich in einer longitudinalen axialen Richtung erstreckt, einen distalen Endabschnitt und einen proximalen Endabschnitt umfasst und dazu eingerichtet ist, in einen Körper eingeführt zu werden; und
einen Betätigungsabschnitt (38), der koaxial mit dem proximalen Endabschnitt des Einführabschnitts (34) verbunden ist, sich in der longitudinalen axialen Richtung erstreckt und dazu eingerichtet ist, von einem Benutzer gehalten und betätigt zu werden, und
wobei der Einführabschnitt (34) einen Biegeabschnitt (36) umfasst, der dazu eingerichtet ist, gebogen zu werden, und
der Betätigungsabschnitt (38) umfasst:
einen ersten Greifabschnitt (40), der sich in der longitudinalen axialen Richtung erstreckt und dazu eingerichtet ist, von einem Benutzer gegriffen zu werden;
einen Biegebetätigungsabschnittskörper (68), der in der longitudinalen axialen Richtung weiter auf einer proximalen Endseite angeordnet ist als der erste Greifabschnitt (40) und einen proximalen Endabschnitt umfasst;
einen Biegebetätigungshebel (46), der in dem Biegebetätigungsabschnittskörper (68) vorgesehen ist, in der longitudinalen axialen Richtung weiter auf einer proximalen Endseite angeordnet ist als der erste Greifabschnitt (40), der dazu eingerichtet ist, den zu biegenden Biegeabschnitt (36) zu betätigen, und einen Stützabschnitt (69) und einen Fingerplatzierungsabschnitt zur Betätigung (71) umfasst, wobei der Stützabschnitt (69) dazu eingerichtet ist, um eine Rotationsachse zu rotieren, die sich eine Breitenrichtung orthogonal zu der longitudinalen axialen Richtung erstreckt und der Fingerplatzierungsabschnitt zur Betätigung (71) mit dem Stützabschnitt (69) gekoppelt ist, wobei sich der Fingerplatzierungsabschnitt zur Betätigung (71) in der Breitenrichtung erstreckt und dazu eingerichtet ist, sich durch eine Rotation des Stützabschnitts (69) entlang der longitudinalen axialen Richtung zu bewegen, wobei ein Daumen und/oder Finger eines Benutzers auf dem Fingerplatzierungsabschnitt zur Betätigung (71) platziert werden kann/können; und
eine Funkeinheit (52), die mit dem proximalen Endabschnitt des Biegebetätigungsabschnittskörpers (68) verbunden und dazu eingerichtet ist, eine Funkkommunikation auszuführen,
**dadurch gekennzeichnet, dass**
die Funkeinheit (52) umfasst:
einen zweiten Greifabschnitt (53), der in der longitudinalen axialen Richtung weiter auf einer proximalen Endseite angeordnet ist als der Biegebetätigungsabschnittskörper (68), sich in der Breitenrichtung erstreckt, dazu eingerichtet ist, von einem Benutzer gegriffen zu werden und ein erstes und ein zweites Greifteil (53a, 53b) umfasst, wobei das erste Greifteil (53a, 53b) in der Breitenrichtung auf einer Seite des zweiten Greifabschnitts (53) vorgesehen ist und in der Breitenrichtung weiter nach außen ragt als der Biegebetätigungshebel (46) und der Biegebetätigungsabschnittskörper (68), das zweite Greifteil (53b, 53a) in der Breitenrichtung auf einer anderen Seite gegenüber der einen Seite des zweiten Greifabschnitts (53) vorgesehen ist und in der Breitenrichtung weiter nach außen ragt als der Biegebetätigungshebel (46) und der Biegebetätigungsabschnittskörper (68) und der gesamte Fingerplatzierungsabschnitt zur Betätigung (71) bezüglich der Breitenrichtung innerhalb der Länge des zweiten Greifabschnitts (53) angeordnet ist; und
eine Antenne (54), die in der Breitenrichtung mit einem Ende des zweiten Greifabschnitts (53) verbunden ist, sich so erstreckt, dass sie distal in der longitudinalen axialen Richtung und nach außen in der Breitenrichtung geneigt ist, nach außen in der Breitenrichtung beabstandet von dem Betätigungsabschnitt (38) angeordnet und dazu eingerichtet ist, ein Funksignal zu übertragen.

2. Endoskop, das dazu eingerichtet ist, mit der Hand gehalten zu werden gemäß Anspruch 1, wobei
das Greifteil (53a, 53b) einen Fingerplatzierungsabschnitt zum Greifen (73) umfasst, der auf einer distalen Seite des Greifteils (53a, 53b) in der longitudinalen axialen Richtung vorgesehen ist und auf dem ein Daumen und/oder Finger eines Benutzers platziert werden kann/können.

3. Endoskop, das dazu eingerichtet ist, mit der Hand gehalten zu werden gemäß Anspruch 1, wobei
der zweite Greifabschnitt (53) eine glatte, gekrümmte Oberflächenform (72) hat.

4. Endoskop, das dazu eingerichtet ist, mit der Hand gehalten zu werden gemäß Anspruch 1, das ferner umfasst:
einen Rotationsmechanismus (51), der dazu eingerichtet ist, die Funkeinheit (52) bezüglich des Biegebetätigungsabschnittskörpers (68) um eine longitudinale Achse zu drehen.

## Revendications

1. Endoscope configuré pour être porté avec une main, comprenant :
une partie d'insertion (34) s'étendant dans une direction longitudinalement axiale, comprenant une partie d'extrémité distale et une partie d'extrémité proximale et configurée pour être insérée dans un corps ; et
une partie d'opération (38) connectée de manière coaxiale à la partie d'extrémité proximale de la partie d'insertion (34), s'étendant dans la direction longitudinalement axiale et configurée pour être tenue et actionnée par un opérateur, et
dans lequel la partie d'insertion (34) comprend une partie de courbure (36) configurée pour être courbée, et
la partie d'opération (38) comprend :
une première partie de préhension (40) s'étendant dans la direction longitudinalement axiale et configurée pour être saisie par un opérateur ;
un corps (68) de partie d'opération de courbure agencé sur un côté d'extrémité davantage proximale dans la direction longitudinalement axiale que la première partie de préhension (40) et comprenant une partie d'extrémité proximale ;
un levier d'opération de courbure (46) prévu dans le corps (68) de partie d'opération de courbure, agencé sur le côté d'extrémité davantage proximale dans la direction longitudinalement axiale que la première partie de préhension (40) configuré pour actionner la partie de courbure (36) destinée à être courbée, et comprenant une partie de support (69) et une partie de placement de doigts pour actionnement (71), dans lequel la partie de support (69) est configurée pour tourner autour d'un axe de rotation s'étendant dans un sens de la largeur orthogonale à la direction longitudinalement axiale et la partie de placement de doigts pour actionnement (71) est couplée à la partie de support (69), dans lequel la partie de placement de doigts pour actionnement (71) s'étend dans le sens de la largeur et est configurée pour se déplacer le long de la direction longitudinalement axiale par une rotation de la partie de support (69), dans lequel au moins l'un parmi un pouce et des doigts d'un opérateur peut être placé sur la partie de placement de doigts pour actionnement (71) ; et
une unité radio (52) connectée à la partie d'extrémité proximale du corps (68) de partie d'opération de courbure et configurée pour réaliser une communication radio,
**caractérisé en ce que**
l'unité radio (52) comprend :
une deuxième partie de préhension (53) agencée sur un côté d'extrémité davantage proximale dans la direction longitudinalement axiale que le corps (68) de partie d'opération de courbure, s'étendant dans le sens de la largeur, configurée pour être saisie par un opérateur et comprenant une première et une deuxième parties de préhension (53a, 53b), dans laquelle la première partie de préhension (53a, 53b) est prévue sur un côté dans le sens de la largeur, de la deuxième partie de préhension (53), et fait saillie vers l'extérieur dans le sens de la largeur plus loin que le levier d'opération de courbure (46) et le corps (68) de partie d'opération de courbure, la deuxième partie de préhension (53b, 53a) est prévue sur l'autre côté dans le sens de la largeur opposé au premier côté, de la deuxième partie de préhension (53), et fait saillie vers l'extérieur dans le sens de la largeur plus loin que le levier d'opération de courbure (46) et le corps (68) de partie d'opération de courbure, et la partie de placement de doigts pour actionnement (71) entière est placée à l'intérieur de la longueur de la deuxième partie de préhension (53), par rapport au sens de la largeur ; et
une antenne (54) connectée à une extrémité dans le sens de la largeur, de la deuxième partie de préhension (53), s'étendant de façon à être inclinée de manière distale dans la direction longitudinalement axiale et vers l'extérieur dans le sens de la largeur, agencée espacée vers l'extérieur dans le sens de la largeur par rapport à la partie d'opération (38) et configurée pour transmettre un signal radio.

2. Endoscope configuré pour être porté avec la main selon la revendication 1, dans lequel
la partie de préhension (53a, 53b) comprend une partie de placement de doigts pour préhension (73) prévue sur un côté distal dans la direction longitudinalement axiale, de la partie de préhension (53a, 53b), et sur laquelle au moins l'un parmi un pouce et des doigts de l'opérateur peut être placé.

3. Endoscope configuré pour être porté avec la main selon la revendication 1, dans lequel
la deuxième partie de préhension (53) présente une forme de surface lisse courbe (72).

4. Endoscope configuré pour être porté avec la main selon la revendication 1, comprenant en outre
un mécanisme de rotation (51) configuré pour faire tourner l'unité radio (52) autour d'un axe longitudinal par rapport au corps (68) de partie d'opération de courbure.
